# DEMANDE DE BREVET EUROPEEN

(11) **EP 1 743 626 A1**
(43) Date de publication de la demande: **17.01.2007**
(21) Numéro de dépôt: 06116037.0
(22) Date de dépôt: 26.06.2006
(51) Int. Cl.: A61K 8/92, A61K 8/81

(54) **Composition de maquillage de la peau comprenant une résine**

(30) Priorité: 13.07.2005 US 698884 P
(71) Demandeur: L'Oreal, 75008 Paris (FR)
(72) Inventeur: Arnaud, Pascal, 94240 L'HAY LES ROSES (FR); Lu, Shaoxiang, Plainsboro, NJ 08536 (US)
(74) Mandataire: Boulard, Denis

(57) **Abrégé**

La présente invention a pour objet une composition de maquillage ou de soin de la peau comprenant une phase grasse liquide comprenant au moins une résine de poids moléculaire moyen en nombre inférieur ou égal à 10000 g/mol, choisie parmi la colophane, les dérivés de colophane, les résines hydrocarbonées et leurs mélanges. L'invention a également pour objet un procédé non thérapeutique de maquillage ou de soin de la peau consistant en l'application sur la peau de ladite composition ainsi que son utilisation pour obtenir un dépôt sur la peau ayant une bonne résistance au transfert, notamment en présence de sébum.

## Description

La présente invention a pour objet une composition de maquillage ou de soin de la peau comprenant une phase grasse liquide et au moins une résine de faible poids moléculaire.

Les compositions cosmétiques de maquillage telles que les fonds de teint, les produits de maquillage du corps, les anticernes, les fards à paupières ou les poudres, comprennent généralement des corps gras tels que des huiles et/ou des cires, et une phase particulaire généralement composée de charges et de pigments. Elles peuvent ainsi se présenter sous la forme d'un gel anhydre, sous forme de stick ou bâton ou sous forme de pâte souple. Elles peuvent encore se présenter sous la forme d'une poudre, qui peut être par exemple libre, compactée ou pressée. Les compositions de maquillage peuvent également comprendre de l'eau ou une phase hydrophile, et se présenter alors notamment sous forme d'émulsion huile-dans-eau, eau-dans-huile, émulsion multiple, notamment lorsqu'il s'agit d'un fond de teint ou de crème teintée.

Les compositions de soin peuvent être en particulier des compositions solaires ou des déodorants.

Les compositions de fond de teint sont couramment employées pour apporter une couleur esthétique à la peau, notamment au visage. Ces produits de maquillage contiennent généralement des huiles, des pigments et/ou charges et éventuellement des additifs comme des actifs cosmétiques ou dermatologiques.

Ces compositions, lorsqu'elles sont appliquées sur la peau, présentent l'inconvénient de transférer, c'est-à-dire de se déposer au moins en partie, en laissant des traces, sur certains supports avec lesquels elles peuvent être mises en contact et notamment un vêtement ou la peau. Il s'ensuit une tenue médiocre du film appliqué, nécessitant de renouveler régulièrement l'application de la composition. Par ailleurs, l'apparition de ces traces inacceptables notamment sur les cols de chemisier peut écarter certaines femmes de l'utilisation de ce type de maquillage.

De plus, le sébum excrété par la peau au cours du temps modifie également les propriétés du maquillage. En particulier, le sébum ne favorise pas l'adhésion du maquillage sur la peau et le transfert du maquillage est encore plus important, engendrant une perte notable du maquillage restant sur la peau.

On recherche donc des compositions de maquillage de la peau « sans transfert » qui présentent l'avantage de former un dépôt résistant au transfert, en particulier en présence de sébum, notamment qui ne se dépose pas, au moins en partie, sur les supports avec lesquels elles sont mises en contact (vêtements, tissus).

La présente invention a donc pour but de fournir une nouvelle voie de formulation d'un produit cosmétique qui permette d'obtenir de bonnes propriétés de résistance au transfert, notamment en présence de sébum.

Or, la Demanderesse a trouvé de manière surprenante qu'en introduisant, dans une composition cosmétique de maquillage ou de soin de la peau, une résine particulière et au moins une huile, il était possible de réaliser un produit de maquillage présentant une très bonne tenue.

La présente invention a pour objet une composition de maquillage ou de soin de la peau comprenant une phase grasse liquide comprenant au moins une résine de poids moléculaire moyen en nombre inférieur ou égal à 10000 g/mol choisie parmi la colophane, les dérivés de colophane, les résines hydrocarbonées et leurs mélanges.

Avantageusement, la composition est apte à former un dépôt ayant un indice de transfert en présence de sébum inférieur ou égal à 3.

L'invention a également pour objet un procédé non thérapeutique de maquillage ou de soin de la peau comprenant l'application sur la peau d'une composition telle que définie précédemment.

L'invention a encore pour objet l'utilisation d'une composition telle que définie précédemment pour obtenir un dépôt, notamment un maquillage, sur la peau ayant une bonne résistance au transfert, notamment en présence de sébum.

### Résine

La résine utilisée dans la composition selon l'invention a de préférence un poids moléculaire moyen en nombre inférieure ou égale à 10 000 g/mol, notamment allant de 250 à 10 000 g/mol de préférence inférieure ou égale à 5 000 g/mol, notamment allant de 250 à 5 000 g/mol, mieux, inférieure ou égale à 2 000 g/mol notamment allant de 250 à 2 000 g/mol et encore mieux inférieure ou égale à 1 000 g/mol notamment allant de 250 à 1 000 g/mol.

On détermine les poids moléculaires moyens en nombre (Mn) par chromatographie liquide par perméation de gel (solvant THF, courbe d'étalonnage établie avec des étalons de polystyrène linéaire, détecteur réfractométrique).

La résine de la composition selon l'invention est avantageusement une résine dite tackifiante. De telles résines sont notamment décrites dans le Handbook of Pressure Sensitive Adhesive, edited by Donatas Satas, 3rd ed., 1989, p. 609-619.

La résine de la composition selon l'invention est choisie parmi la colophane, les dérivés de colophane, les résines hydrocarbonées et leurs mélanges.

La colophane est un mélange comprenant majoritairement des acides organiques appelés acides de colophane (principalement des acides de type abiétique et de type pimarique).
Il existe trois types de colophane : la colophane ("gum rosin") obtenue par incision sur les arbres vivants, la colophane de bois ("wood rosin") qui est extraite des souches ou du bois de pins, et l'huile de tall ("tall oil rosin") qui est obtenue d'un sous-produit provenant de la production du papier.

Les dérivés de colophane peuvent être issus en particulier de la polymérisation, de l'hydrogénation et/ou de l'estérification (par exemple avec des alcools polyhydriques tels que l'ethylène glycol, le glycérol, le pentaérhytritol) des acides de colophanes. On peut citer par exemple les esters de colophanes commercialisés sous la référence FORAL 85, PENTALYN H et STAYBELITE ESTER 10 par la société HERCULES ; SYLVATAC 95 et ZONESTER 85 par la société ARIZONA CHEMICAL ou encore UNIREZ 3013 par la société UNION CAMP.

Les résines hydrocarbonées sont choisies parmi les polymères de faible poids moléculaire qui peuvent classifiées, selon le type de monomère qu'elles comprennent, en :
- résines hydrocarbonées indéniques telles que les résines issues de la polymérisation en proportion majoritaire de monomère indène et en proportion minoritaire de monomère choisi parmi le styrène, le méthylindène, le méthylstyrène et leurs mélanges. Ces résines pouvant éventuellement être hydrogénées. Ces résines peuvent présenter un poids moléculaire allant de 290 à 1150 g/mol.
   Comme exemples de résines indéniques, on peut citer celles commercialisées sous la référence ESCOREZ 7105 par la société Exxon Chem., NEVCHEM 100 et NEVEX 100 par la société Neville Chem., NORSOLENE S105 par la société Sartomer, PICCO 6100 par la société Hercules et RESINALL par la société Resinall Corp., ou les copolymères indène/méthylstyrène/styrène hydrogéné commercialisées sous la dénomination « REGALITE » par la société Eastman Chemical, en particulier REGALITE R 1100, REGALITE R 1090, REGALITE R-7100, REGALITE R1010 HYDROCARBON RESIN, REGALITE R1125 HYDROCARBON RESIN.
- les résines aliphatiques de pentanediène telles que celle issues de la polymérisation majoritairement du monomères 1,3-pentanediène (trans ou cis pipérylène) et de monomère minoritaire choisi parmi l'isoprène, le butène, le 2-méthyl-2-butène, le pentène, le 1,4-pentanediène et leurs mélanges. Ces résines peuvent présenter un poids moléculaire allant de 1000 à 2500 g/mol.
   De telles résines du 1,3-pentanediène sont commercialisées par exemple sous les références PICCOTAC 95 par la société Eastman Chemical, ESCOREZ 1304 par la société Exxon Chemicals., NEVTAC 100 par la société Neville Chem. ou WINGTACK 95 par la société Goodyear;
- les résines mixtes de pentanediène et d'indène, qui sont issues de la polymérisation d'un mélange de monomères de pentanediène et d'indéne tels que ceux décrits ci-dessus, comme par exemple les résines commercialisées sous la référence ESCOREZ 2101 par la société Exxon Chemicals., NEVPENE 9500 par la société Neville Chem., HERCOTAC 1148 par la société Hercules., NORSOLENE A 100 par la société Sartomer, WINGTACK 86, WINGTACK EXTRA et WINGTACK PLUS par la société Goodyear,
- les résines diènes des dimères du cyclopentanediène telles que celles issues de la polymérisation de premier monomère choisi parmi l'indène et le styrène, et de deuxième monomère choisi parmi les dimères du cyclopentanediène tels que le dicyclopentanediène, le méthyldicyclopentanediène, les autres dimères du pentanediène, et leurs mélanges. Ces résines présentent généralement un poids moléculaire allant de 500 à 800 g/mol, telles que par exemple celles commercialisées sous la référence BETAPRENE BR 100 par la société Arizona Chemical Co., NEVILLE LX-685-125 et NEVILLE LX-1000 par la société Neville Chem., PICCODIENE 2215 par la société Hercules, PETRO-REZ 200 par la société Lawter ou RESINALL 760 par la société Resinall Corp. ;
- les résines diènes des dimères de l'isoprène telles que les résines terpèniques issues de la polymérisation d'au moins un monomère choisi parmi α-pinène, le β-pinène, le limonène, et leurs mélanges. Ces résines peuvent présenter un poids moléculaire allant de 300 à 2000 g/mol. De telles résines sont commercialisées par exemple sous la dénomination PICCOLYTE A115 et S125 par la société Hercules, ZONAREZ 7100 ou ZONATAC 105 LITE par la société ARIZONA Chem.
   On peut également citer certaines résines modifiées telles que les résines hydrogénées comme celles commercialisées sous la dénomination EASTOTAC C6-C20 POLYOLEFINE par la société Eastman Chemical Co., sous la référence ESCOREZ 5300 par la société Exxon Chemicals ou encore les résines NEVILLAC HARD ou NEVROZ proposées par la société Neville Chem., les résines PICCOFYN A-100, PICCOTEX 100 ou PICCOVAR AP25 proposées par la société Hercules ou la résine SP-553 proposée par société Schenectady Chemical Co.

Selon un mode préféré de réalisation, la résine est choisie parmi résines hydrocarbonées indéniques en particulier les copolymères indène/méthylstyrène/styrène hydrogéné commercialisées sous la dénomination « REGALITE » par la société Eastman Chemical, tels que la REGALITE R 1100, REGALITE R 1090, REGALITE R-7100, REGALITE R1010 HYDROCARBON RESIN, REGALITE R1125 HYDROCARBON RESIN.

La résine peut être présente dans la composition selon l'invention en une teneur allant de 0,1 à 30 % en poids, par rapport au poids total de la composition, de préférence allant de 0,3 à 15 % en poids, plus préférentiellement allant de 0,5 à 10 % en poids.

### Phase grasse

La composition selon l'invention comprend une phase grasse liquide.

La phase grasse liquide comprend au moins une huile choisie parmi les huiles volatiles, les huiles non volatiles, et leurs mélanges.

Selon un mode préféré de réalisation, la composition selon l'invention comprend au moins une huile volatile.

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

Selon un mode préféré de réalisation, la composition selon l'invention comprend au moins une huile volatile hydrocarbonée.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'lsopars^{®} ou de Permethyls^{®}.

Comme huiles volatiles, on peut aussi utiliser les silicones volatiles, comme par exemple les huiles de silicones linéaires ou cycliques volatiles, notamment celles ayant une viscosité ≤ 5 centistokes (5 x 10⁻⁶ m²/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone.

Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile fluorée n'a généralement pas de point éclair.
Comme huile volatile fluorée, on peut citer le nonafluoroéthoxybutane, le nonafluorométhoxybutane, le décafluoropentane, le tétradécafluorohexane, le dodécafluoropentane, et leurs mélanges.

La composition selon l'invention comprend une huile volatile en une teneur allant de 0,1à 99% en poids par rapport au poids total de la composition, de préférence de 2 à 80 % en poids, et plus préférentiellement allant de 5 à 70 % en poids.

La composition selon l'invention peut comprendre au moins une huile non volatile.

Par "huile non volatile", on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810^{®}, 812^{®} et 818^{®} par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam ®, le squalane, les huiles de paraffine, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

La phase grasse liquide peut être présente dans la composition selon l'invention en une teneur totale allant de 0,5 % à 98 % en poids, par rapport au poids total de la composition, de préférence allant de 2 % à 50 % en poids, de préférence allant de 5 % à 40 % en poids.

La phase grasse de la composition selon l'invention peut également comprendre des corps gras autres que les huiles citées ci-dessus, comme des cires ou encore des corps gras pâteux.

Par cires on entend un corps gras solide à température ambiante.

On peut définir les corps gras pâteux à l'aide d'au moins une des propriétés physico-chimiques suivantes :
- une viscosité de 0,1 à 40 Pa.s (1 à 400 poises), mesurée à 40 °C avec un viscosimètre rotatif CONTRAVES TV équipé d'un mobile MS-r3 ou MS-r4 à la fréquence de 60 Hz,
- un point de fusion de 25-70 °C, de préférence 25-55 °C.

A titre de cires pouvant être utilisées selon l'invention, on peut citer :
- les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre,
- les cires minérales, par exemple de paraffine, de vaseline, de lignite ou les cires microcristallines ou les ozokérites,
- les cires synthétiques parmi lesquelles les cires de polyéthylène, et les cires obtenues par synthèse de Fisher-Tropsch,
- les cires de silicone, en particulier les polysiloxanes linéaires substitués; on peut citer, par exemple, les cires de silicone polyéther, les alkyl ou alkoxy-diméthicones ayant de 16 à 45 atomes de carbone, les alkyl méthicones comme la C₃₀-C₄₅ alkyl méthicone vendue sous la dénomination commerciale "AMS C 30" par DOW CORNING,
- les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C₂₀-C₄₀ vendu sous la dénomination commerciale "KESTER WAX K82H" par la société KOSTER KEUNEN,
- et/ou leurs mélanges.

De préférence, on utilisera les cires de polyéthylène, les cires microcristallines, les cires de carnauba, l'huile de jojoba hydrogénée, les cires de candellila, les cires d'abeilles et/ou leurs mélanges.

De préférence, les cires sont présentes à une teneur allant de 0,05 à 30% en poids, de préférence encore de 0,1 à 20 %, par rapport au poids total de la composition.

Ces corps gras peuvent en particulier être choisis de manière variée par l'homme du métier afin de préparer une composition ayant les propriétés souhaitées, par exemple en consistance ou en texture.

### Copolymère bloc hydrocarboné

Selon un mode de réalisation de l'invention, la composition peut comprendre, outre la résine, un copolymère séquencé hydrocarboné appelé également copolymère bloc, de préférence un copolymère séquencé soluble dans une phase grasse liquide telle que définie précédemment.

Le copolymère peut présenter au moins un bloc dont la température de transition vitreuse, est de préférence inférieure à 20°C, de préférence inférieure ou égale à 0°C, de préférence inférieure ou égale à -20°C, de préférence encore inférieure ou égale à - 40°C. La température de transition vitreuse dudit bloc peut être comprise entre -150°C et 20°C, notamment entre -100°C et 0°C.
Dans ce cas, lorsque la résine est dotée d'au moins une température de transition vitreuse, l'écart entre les températures de transition vitreuse de la résine et du copolymère est généralement supérieur à 20°C, de préférence supérieur à 40°C, et mieux supérieur à 60°C.
Lorsque la résine est dotée d'au moins une température de transition vitreuse, le copolymère séquencé est avantageusement un plastifiant de la résine décrite précédemment. On entend par plastifiant de la résine, un composé, qui associé en quantité suffisante à la résine, abaisse la température de transition vitreuse de la résine telle que définie précédemment. Le composé plastifiant abaisse notamment la température de transition vitreuse du polymère d'au moins 2, 3 ou 4 °C, de préférence de 5°C à 20°C. Dans un mode de réalisation préféré, le composé plastifiant abaisse notamment la température de transition vitreuse du polymère d'au moins 2, 3 ou 4 °C, de préférence de 5°C à 20°C.

On préfère notamment les copolymères séquencés comprenant au moins un bloc styrène et au moins un bloc comprenant des motifs choisis parmi le butadiène, l'éthylène, le propylène, le butylène, l'isoprène ou un de leurs mélanges.

Le copolymère bloc hydrocarboné peut être notamment un copolymère dibloc, tribloc, multibloc, radial, étoile, ou leurs mélanges.
De tels copolymères blocs hydrocarbonés sont décrits dans la demande US-A-2002/005562 et dans le brevet US-A-5 221 534. Selon un mode particulièrement préféré de réalisation, l'agent structurant des huiles est un polymère lipophile.

Le copolymère bloc hydrocarboné présent dans la composition selon l'invention est un copolymère amorphe formé par polymérisation d'une oléfine. L'oléfine peut être notamment un monomère à insaturation éthylénique élastomérique.
Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

Avantageusement, le copolymère bloc hydrocarboné est un copolymère bloc amorphe de styrène et d'oléfine.

Selon un mode préféré de réalisation, le copolymère bloc hydrocarboné est hydrogéné pour réduire les insaturations éthyléniques résiduelles après la polymérisation des monomères.

En particulier, le copolymère bloc hydrocarboné est un copolymère, éventuellement hydrogéné, à blocs styrène et à blocs éthylène/alkylène en C₃-C₄.

Comme copolymère dibloc, de préférence hydrogéné, on peut citer les copolymères de styrène-éthylène/propylène, les copolymères de styrène-éthylène/butadiène, les copolymères de styrène-éthylène/butylène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-éthylène/butylène-styrène les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

Selon un mode de réalisation de la présente invention, le copolymère bloc hydrocarboné est un copolymère tribloc styrène-éthylène/butylène-styrène.

Selon un mode préféré de réalisation de l'invention, on peut notamment utiliser un mélange d'un copolymère tribloc styrène-butylène/éthylène-styrène et d'un copolymère dibloc styrène-éthylène/butylène, notamment ceux vendus sous la dénomination Kraton® G1657M par la société Kraton Polymers.

On peut également utiliser un mélange de copolymère hydrogéné tribloc styrène-butylène/éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL^{®} M5960 et VERSAGEL^{®} M5670.

L'invention a également pour objet une composition de maquillage ou de soin de la peau comprenant une phase grasse liquide telle que définie précédemment, comprenant au moins une résine de poids moléculaire moyen en nombre inférieur ou égal à 10000 g/mol, choisie parmi la colophane, les dérivés de colophane, les résines hydrocarbonées et leurs mélanges tel que défini précédemment, et au moins un copolymère bloc hydrocarboné tel que défini précédemment, la composition étant apte à former un dépôt ayant un indice de transfert en présence de sébum inférieur ou égal à 3.

Selon un mode préféré de réalisation, la composition selon l'invention comprend une phase grasse liquide telle que définie précédemment, une résine de poids moléculaire moyen en nombre inférieur ou égal à 10000 g/mol, choisie parmi la colophane, les dérivés de colophane, les résines hydrocarbonées et leurs mélanges, et au moins un copolymère bloc hydrocarboné choisi parmi les copolymères dibloc de styrène-éthylène/propylène, de styrène-éthylène/butadiène, et les copolymères tribloc de styrène-éthylène/butadiène-styrène, de styrène-isoprène-styrène et de styrène-butadiène-styrène, le rapport pondéral de la résine sur le copolymère bloc hydrocarboné allant de 1/1 à 4/1.

De préférence, le rapport pondéral de la résine sur le copolymère bloc hydrocarboné va de 2,5/1 à 3,5/1

Selon un mode préféré de réalisation, la résine est choisie parmi les copolymères indène/méthylstyrène/styrène hydrogéné.

Le copolymère bloc hydrocarboné (ou le mélange de copolymères blocs hydrocarbonés) peut être présent en une teneur allant de 0,1 % à 10 % en poids, par rapport au poids total de la composition, de préférence allant de 0,5 % à 5 % en poids, et plus préférentiellement allant de 1 % à 3 % en poids.

Selon un mode particulier de réalisation, le copolymère bloc hydrocarboné est présent dans la composition selon l'invention, en une teneur telle que le rapport pondéral de la résine sur le copolymère bloc hydrocarboné va de 1/1 à 4/1.

Selon un mode préféré de réalisation, le rapport pondéral de la résine sur le copolymère bloc hydrocarboné va de 2,5/1 à 3,5/1.

### Agent épaississant des huiles additionnels:

La composition selon l'invention peut comprendre, outre le copolymère bloc hydrocarboné, au moins un agent épaississant des huiles choisi parmi les agents épaississants polymériques, les agents épaississants minéraux et leurs mélanges.

L'agent épaississant polymérique des huiles présent dans la composition selon l'invention peut être un polymère amorphe formé par polymérisation d'une oléfine. L'oléfine peut être notamment un monomère à insaturation éthylénique élastomérique. Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

L'agent épaississant polymérique des huiles est capable d'épaissir ou de gélifier la phase organique de la composition. Par polymère amorphe, on entend un polymère qui n'a pas de forme cristalline. L'agent épaississant polymérique peut être également filmogène, c'est-à-dire qu'il est capable de former un film lors de son application sur la peau.
L'agent épaississant polymérique des huiles peut notamment être choisi parmi:
- les polycondensats de type polyamide résultant de la condensation entre (α) au moins un acide choisi parmi les acides dicarboxyliques comprenant au moins 32 atomes de carbone tels que les acides gras dimères et (β) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG^{®} par la société ARIZONA CHEMICAL ;
- les polymères siliconés du type :
   1) des polyorganosiloxanes comportant au moins deux groupes capables d'établir des interactions hydrogène, ces deux groupes étant situés dans la chaîne du polymère, et/ou
   2) des polyorganosiloxanes comportant au moins deux groupes capables d'établir des interactions hydrogène, ces deux groupes étant situés sur des greffons ou ramifications.
   Les groupes capables d'établir des interactions hydrogène peuvent être choisis parmi les groupes ester, amide, sulfonamide, carbamate, thiocarbamate, urée, uréthane, thiourée, oxamido, guanidino, biguanidino et leurs combinaisons.

Les polymères siliconés utilisés comme agents structurants dans la composition de l'invention sont des polymères du type polyorganosiloxane comme par exemple ceux décrits dans les documents US-A-5 874 069, US-A-5,919,441, US-A-6,051,216 et US-A-5,981,680.

En particulier, les polymères siliconés sont des polyorganosiloxanes tels que définis ci-dessus et dont les motifs capables d'établir des interactions hydrogènes sont disposés dans la chaîne du polymère.
Les polymères siliconés peuvent plus particulièrement être des polymères comprenant au moins un motif répondant à la formule générale I: 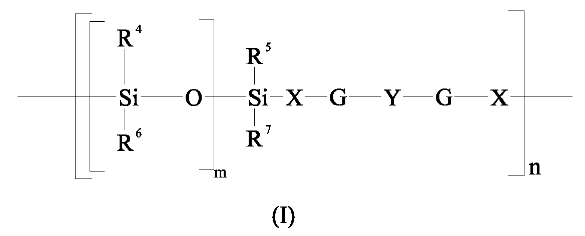 dans laquelle :
1) R⁴, R⁵, R⁶ et R⁷, identiques ou différents, représentent un groupe choisi parmi :
   - les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
   - les groupes aryles en C₆ à C₁₀, éventuellement substitués par un ou plusieurs groupes alkyle en C₁ à C₄,
   - les chaînes polyorganosiloxanes contenant ou non un ou plusieurs atomes d'oxygène, de soufre et/ou d'azote,
2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
4) Y représente un groupe répondant à la formule : 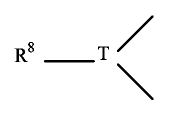 dans laquelle
   - T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S,
   ou T représente un atome trivalent choisi parmi N, P et Al, et
   - R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
5) les G, identiques ou différents, représentent les groupes divalents choisis parmi : 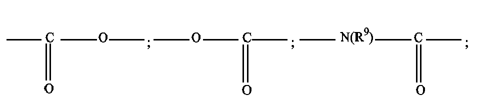 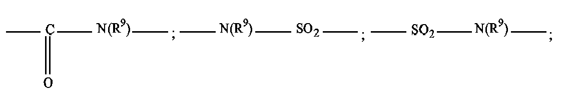 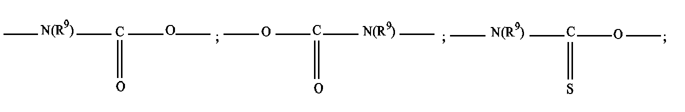 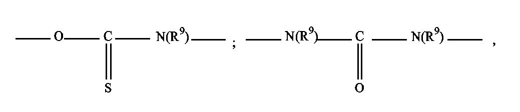 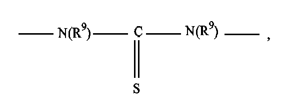 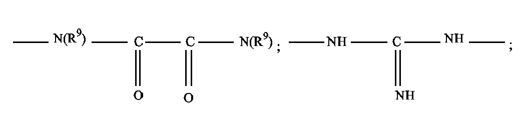 et 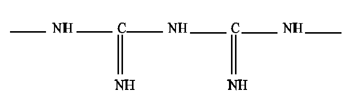 où R⁹ représente un atome d'hydrogène ou un groupe alkyle, linéaire ou ramifié, en C₁ à C₂₀, à condition qu'au moins 50 % des R⁹ du polymère représente un atome d'hydrogène et qu'au moins deux des groupes G du polymère soient un autre groupe que : 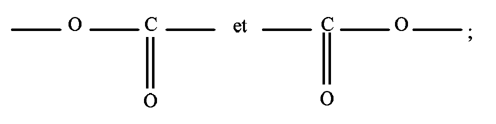
6) n est un nombre entier allant de 2 à 500, de préférence de 2 à 200, et m est un nombre entier allant de 1 à 1000, de préférence de 1 à 700 et mieux encore de 6 à 200.
   Selon l'invention, 80 % des R⁴, R⁵, R⁶ et R⁷, du polymère sont choisis de préférence parmi les groupes méthyle, éthyle, phényle et 3,3,3-trifluoropropyle.
   Selon un mode de réalisation avantageux, les groupes capables d'établir des interactions hydrogènes sont des groupes amides de formule ―C(O)NH- et ―HN-C(O)-.
   Dans ce cas, l'agent structurant peut être un polymère comprenant au moins un motif de formule (III) ou (IV) : 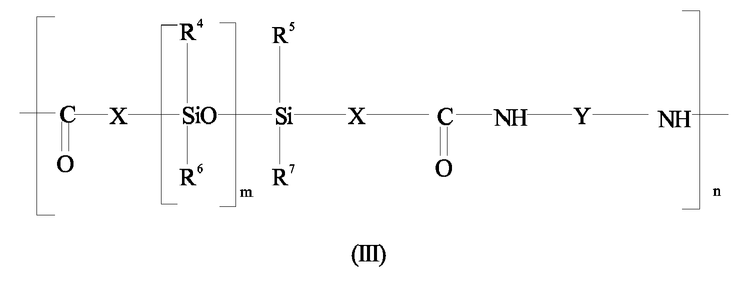
ou 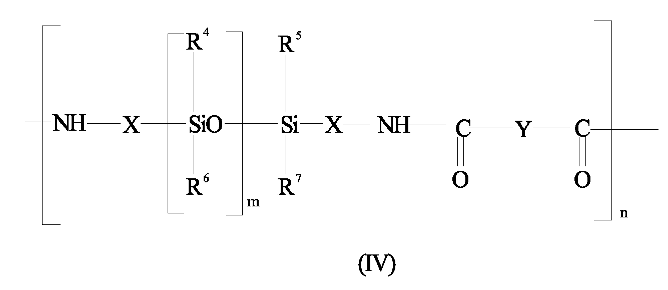 dans lesquelles R⁴, R⁵, R⁶, R⁷, X, Y, m et n sont tels que définis ci-dessus.
Dans ces polyamides de formule (III) ou (IV), m va de 1 à 700, en particulier de 15 à 500 et notamment de 50 à 200 et n va particulier de 1 à 500, de préférence de 1 à 100 et mieux encore de 4 à 25,
- X est de préférence une chaîne alkylène linéaire ou ramifiée ayant de 1 à 30 atomes de carbone, en particulier 1 à 20 atomes de carbone, notamment de 5 à 15 atomes de carbone et plus particulièrement de 10 atomes de carbone, et
- Y est de préférence une chaîne alkylène linéaire ou ramifiée ou pouvant comporter des cycles et/ou des insaturations, ayant de 1 à 40 atomes de carbone, en particulier de 1 à 20 atomes de carbone, et mieux encore de 2 à 6 atomes de carbone, en particulier de 6 atomes de carbone.
- les galactommananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges ;

La composition selon l'invention peut également comprendre au moins un agent épaississant minéral des huiles tel qu'une argile organophile, les silices pyrogénées.

Les argiles organophiles sont des argiles modifiées par des composés chimiques rendant l'argile apte à gonfler dans les milieux huileux.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.
Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques comme la peau.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130^{®}", "AEROSIL 200^{®}", "AEROSIL 255^{®}", "AEROSIL 300^{®}", "AEROSIL 380^{®}" par la société Degussa, "CAB-O-SIL HS-5^{®}", "CAB-O-SIL EH-5^{®}", "CAB-O-SIL LM-130^{®}", "CAB-O-SIL MS-55^{®}", "CAB-O-SIL M-5^{®}" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812^{®}" par la société Degussa, "CAB-O-SIL TS-530^{®}" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972^{®}", "AEROSIL R974^{®}" par la société Degussa, "CAB-O-SIL TS-610^{®}", "CAB-O-SIL TS-720^{®}" par la société Cabot.

La silice pyrogénée présente de préférence une taille de particules pouvant être nanométrique à micrométrique, par exemple allant d'environ de 5 à 200 nm.

L'agent épaississant minéral des huiles peut être présent dans la composition selon l'invention en une teneur allant de 0,5 % à 7 % en poids, par rapport au poids total de la composition, de préférence en une teneur allant de 1 % à 5 % en poids, et préférentiellement allant de 1 % à 3 % en poids.

L'agent épaississant des huiles est présent dans la composition selon l'invention en une teneur totale allant de 0,1 à 20% en poids par rapport au poids total de la composition, de préférence allant de 0,5 à 15 % en poids, et plus préférentiellement allant de 1 à 10% en poids.

### Phase aqueuse

La composition selon l'invention peut comprendre une phase aqueuse.

La phase aqueuse comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants organiques miscibles à l'eau (à température ambiante - 25 °C) comme par exemple les monoalcools ayant de 2 à 6 atomes de carbone tels que l'éthanol, l'isopropanol ;
les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol, l'hexylène glycol, le dipropylène glycol, le diéthylène glycol ;
les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol, et leurs mélanges.

La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensioactifs et leurs mélanges.

De préférence, la phase aqueuse peut être présente dans la composition selon l'invention en une teneur allant de 1 à 95 % en poids, par rapport au poids total de la composition, et de préférence de 3 à 80 % en poids, et plus préférentiellement de 5 à 60 % en poids.

### Phase pulvérulente

La composition selon l'invention peut comprendre une phase pulvérulente notamment choisie parmi les pigments, les charges et/ou les nacres et leurs mélanges.

Selon un mode préféré de réalisation, la composition selon l'invention peut comprendre des pigments.

Par « pigments », il faut comprendre des particules, minérales ou organiques, insolubles dans la phase organique liquide, destinées à colorer et/ou opacifier la composition.

Les pigments peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique, l'oxychlorure de bismuth, la nacre, le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.
On utilise de préférence des pigments d'oxydes de fer ou de dioxyde de titane.

Les pigments peuvent être traités avec un agent hydrophobe pour les rendre compatible avec la phase organique de la composition. L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl si lanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.
Des pigments traités hydrophobes sont notamment décrits dans la demande EP-A-1086683.

Les pigments peuvent être présents, dans la composition selon l'invention, en une teneur supérieure ou égale à 0,01 à 50% en poids, par rapport au poids total de la composition, en particulier allant de 0,1% à 30% en poids, préférentiellement allant de 0,5% à 20% en poids, en particulier allant de 0,5 % à 15% en poids.

Outre les pigments, la phase pulvérulente de la composition selon l'invention peut comprendre des charges et/ou des nacres.

Selon un mode préféré de réalisation, la composition selon l'invention peut comprendre des charges.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®), les poudres de poly-β-alanine, les poudres de polyéthylène, les polyméthacrylates de métyle, les poudres de polyuréthane telle que la poudre de copolymère de diisocyanate d'hexaméthylène et de triméthylol hexyl lactone vendue sous les dénominations PLASTIC POWDER D-400 par la société TOSHIKI, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les poudres de résine de silicone, en particulier les poudres de silsesquioxane (poudres de résine de silicone notamment décrites dans le brevet EP 293795 ; Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; le sulfate de baryum et leurs mélanges.

Les charges peuvent être présentes dans la composition selon l'invention en une teneur totale allant de 0,01 % à 99 % en poids, par rapport au poids total de la composition, de préférence allant de 0,02 % à9 % en poids, et préférentiellement allant de 0,05 % à 90 % en poids.

Outre les pigments et les charges, la phase particulaire de la composition selon l'invention peut comprendre des nacres.

Par « nacres », il faut comprendre des particules irisées, notamment produites par certains mollusques dans leur coquille ou bien synthétisées, insolubles dans le milieu de la composition.

Les nacres peuvent être choisies parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les nacres peuvent être présents la composition selon l'invention en une teneur allant de 0,01 % à 50 % en poids, par rapport au poids total de la composition, de préférence allant de 0,02 % à 30 % en poids, et préférentiellement allant de 0,5 % à 20 % en poids.

### Colorants additionnels

La composition selon l'invention peut comprendre des colorants additionnels choisis parmi les colorants hydrosolubles et liposolubles.

Les colorants hydrosolubles sont par exemple le jus de betterave, le bleu de méthylène, le caramel.

Par colorants liposolubles, il faut comprendre des composés généralement organiques solubles dans les corps gras comme les huiles.
Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.

Les colorants additionnels peuvent être présents la composition selon l'invention en une teneur allant de 0,001 % à 30 % en poids, par rapport au poids total de la composition, de préférence allant de 0,01 % à 20 % en poids, et préférentiellement allant de 0,02 % à 10 % en poids.

### Ingrédients cosmétiques usuels additionnels

La composition selon l'invention peut comprendre au moins un autre ingrédient cosmétique usuel pouvant être choisi notamment parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les agents déodorants, les sequestrants, les agents filmogènes, et leurs mélanges.

L'invention a également pour objet, selon un autre aspect, un procédé de maquillage et/ou de soin de la peau consistant à appliquer sur le peau une composition comprenant une phase grasse liquide comprenant une résine de poids moléculaire moyen en nombre inférieur ou égal à 10000 g/mol, choisie parmi la colophane, les dérivés de colophane, les résines hydrocarbonées et leurs mélanges, et au moins un copolymère bloc hydrocarboné choisi parmi les copolymères dibloc de styrène-éthylène/propylène, de styrène-éthylène/butadiène, et les copolymères tribloc de styrène-éthylène/butadiène-styrène, de styrène-isoprène-styrène et de styrène-butadiène-styrène, le rapport pondéral de la résine sur le copolymère bloc hydrocarboné allant de 1/1 à 4/1.

Selon un mode préféré de réalisation, le rapport pondéral de la résine sur le copolymère bloc hydrocarboné de la composition du procédé de maquillage et/ou de soin va de 2,5/1 à 3,5/1.

Selon un mode préféré de réalisation, la résine de la composition du procédé de maquillage et/ou de soin est choisie parmi les copolymères indène/méthylstyrène/styrène hydrogéné.

### Mesure de l'indice de transfert

L'indice de transfert en présence de sébum du dépôt obtenu avec la composition selon l'invention est déterminé selon le protocole de mesure décrit ci-après.

On prépare un support (carré de 40 mm X 40 mm) constitué d'une couche de mousse de néoprène adhésif sur une de ses faces (vendue sous la dénomination RE70X40 212B de la société JOINT TECHNIQUE LYONNAIS IND). Sur la face non adhésive du support on fixe une couronne adhésive ayant un diamètre interne de 24 mm et dont l'épaisseur est d'environ 250 µm. On applique à l'intérieur de la couronne la composition que l'on arase avec une lame de verre pour obtenir un dépôt de la composition d'environ 250 µm d'épaisseur puis on retire la couronne et on laisse sécher pendant 20 heures à l'étuve à 37 °C.

Le support est ensuite collé par sa face adhésive sur un embout d'un diamètre de 27 mm fixé sur une presse (STATIF MANUEL SV-1 de la société IMADA Co LTD) équipée d'un dynanomètre (DPS-5R de la société IMADA Co LTD).

Sur un papier couché qualité photo (référence EPSON S041061 de 102g/m²), on dessine une bande de 4 cm de largeur et 21 cm de longueur et dans cette bande on dessine 5 cases ayant chacune une longueur de 4,2 cm selon l'axe longitudinal de la bande. Le papier est placé sur le socle de la presse.

On dépose au centre de la première case une goutte de 10 µl de sébum artificiel ayant la composition suivante :
- trioléine 29 %
- acide oléïque 28,5 %
- oléate d'oléyle 18,5%
- squalène 14 %
- cholestérol 7 %
- palmitate de cholestérol 3 %

Puis on presse le support (comportant l'échantillon de composition) sur la première case de la bande de papier à une force d'environ 4 kg exercée pendant 5 secondes. Puis on déplace de manière rectiligne et régulière le papier sur toute la longueur de la bande de telle sorte que le support soit en contact avec toute la longueur de la bande. La vitesse de déplacement de la bande est de l'ordre de 10 cm/s.

On observe alors visuellement la traînée de produit déposée sur la bande de papier. On attribue une note allant de 0 à 5 par incréments de 0,5 en fonction du nombre de cases, de la première à la cinquième, traversées en tout ou partie par l'éventuelle traînée de produit.

Pour certains produits, non colorés, une étape de révélation peut être nécessaire de manière à rendre visible la traînée de produit. A titre d'exemple on utilise un composé apte à produire une réaction colorée au contact du produit transféré. Selon un autre exemple, on incorpore au produit à tester un actif qui émet dans le visible au moins une partie d'un rayonnement UV (lampe de Wood).

La note 5 est attribuée lorsqu'à l'observation, après avoir fait le déplacement relatif entre le papier et le support, il ne subsiste sur le support sensiblement aucun produit (moins de 10%). Dans ce dernier cas, le transfert peut être qualifié de total.

La note 5 est également attribuée lorsque la traînée de produit s'étend au delà de la cinquième case, indépendamment de la quantité de produit subsistant sur le support.

La note 0 est attribuée dans le cas où aucun produit présent sur le support n'est transféré sur la bande de papier. Aucune trace visible ne peut être observée sur la feuille. Le transfert peut être qualifié de nul.

Par convention, le trait de séparation entre la case n et la case n+1 fait partie de la case n.

Le tableau ci dessous illustre la façon dont sont attribuées les autres notes en fonction de l'endroit sur les cases 1 à 5 où s'arrête la traînée de produit. Pour ces notes, il reste une quantité plus ou moins grande de produit sur le support. Le transfert est partiel.

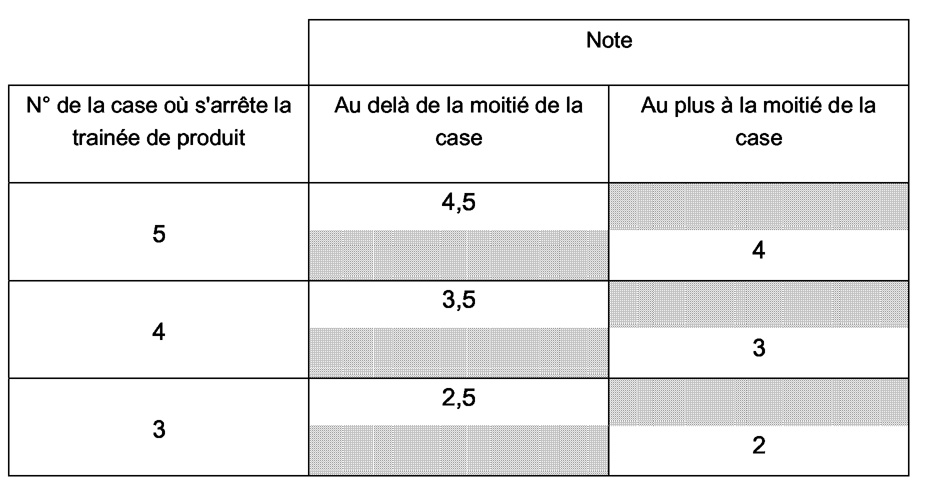

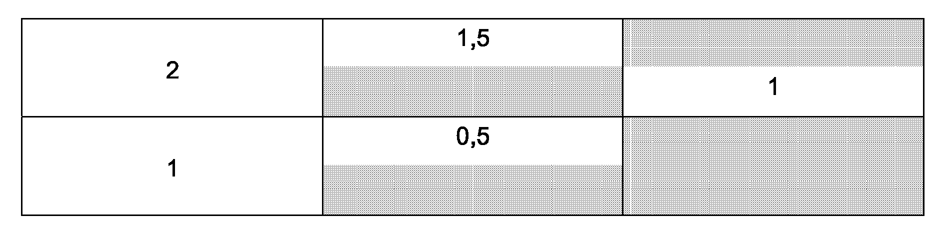

La composition selon l'invention peut former un dépôt ayant un indice de transfert en présence de sébum inférieur ou égal à 3, de préférence inférieur ou égal à 2 et plus préférentiellement inférieur ou égal à 1,5.

L'invention est présentée plus en détail dans les exemples ci-après.

### Exemples 1 à 7 :

On a préparé 7 fonds de teint sous forme d'émulsion eau-dans-huile ayant la formule générale suivante :

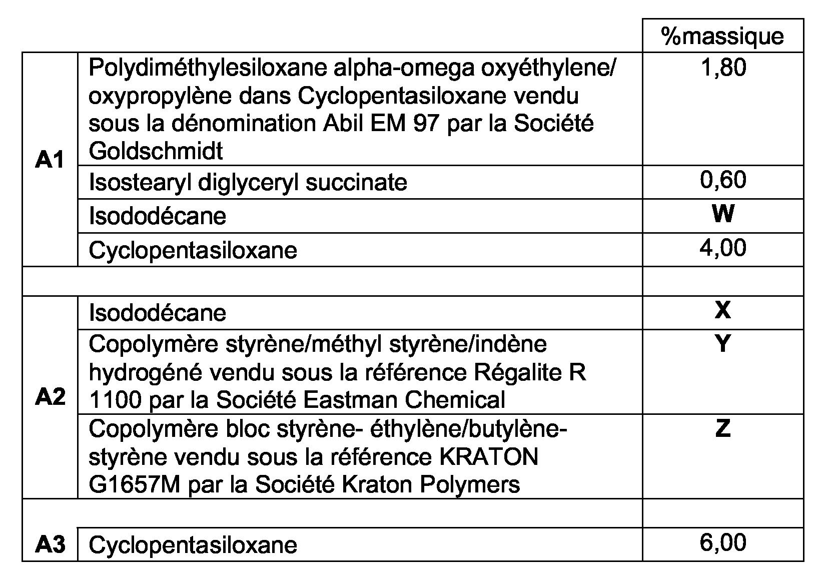

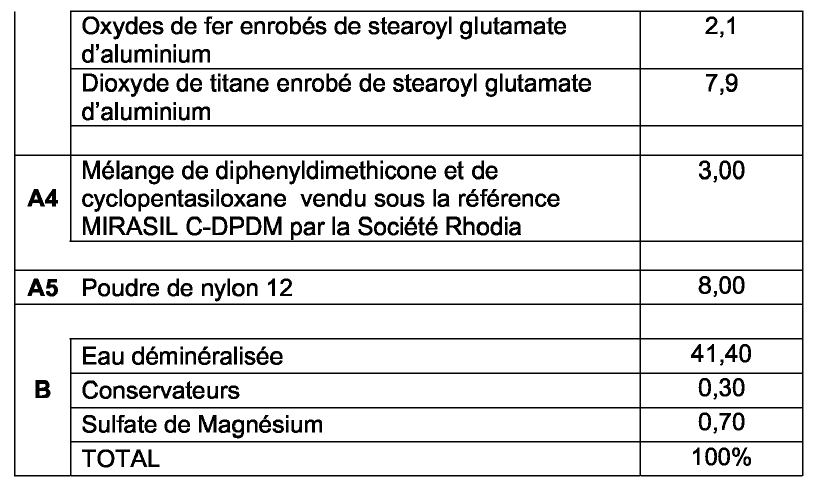

Le taux massique du copolymère styrène/méthyl styrène/indène hydrogéné et du copolymère bloc styrène- éthylène/butylène- styrène (Régalite R 1100 + KRATON G1657M) dans la composition est fixé à Y+Z= 2,1 %.

Le taux massique d'isododécane est constant dans la composition, soit W+X= 22,1 %. Une fraction de cet isododécane est utilisée pour solubiliser les deux polymères.

On fait varier le rapport pondéral de copolymère styrène/méthyl styrène/indène hydrogéné / copolymère bloc styrène- éthylène/butylène- styrène (Régalite R 1100 + KRATON G1657M).

| | **Exemple 1** | **Exemple 2** | **Exemple 3** | **Exemple 4** | **Exemple 5** | **Exemple 6** | **Exemple 7** |
|---|---|---|---|---|---|---|---|
| W (Isododécane A1) | 11,07 | 15,8 | 14,20 | 10,2 | 16,12 | 17,43 | 3,2 |
| X (Isododécane A2) | 11,03 | 6,3 | 7,90 | 11,9 | 5,98 | 4,67 | 18,9 |
| Y Régalite R 1100 | 2,1 | 1,68 | 1,60 | 1,4 | 1,3 | 1,08 | 1,05 |
| Z Kraton G1657M | 0 | 0,42 | 0,50 | 0,7 | 0,8 | 1,02 | 1,05 |
| | | | | | | | |
| % Régalite R 1100 | 100 | 80,00 | 76,19 | 66,67 | 61,90 | 51,43 | 50,00 |
| % Kraton G1657M | 0 | 20,00 | 23,81 | 33,33 | 38,10 | 48,57 | 50,00 |
| Ratio Régalite R 1100 / Kraton G1657M | | 4 | 3,2 | 2 | 1,62 | 1,06 | 1 |

### 2) Mode opératoire

### - Préparation des phases:

* Préparation de A2 : dans un poêlon disperser la Régalite R 1100 dans l'isododécane à 90°C puis ajouter le Kraton G1657M, attendre la dispersion totale pour arrêter le chauffage et l'agitation.
* Broyer les pigments à la tricylindre (3 passages) dans le cyclopentasiloxane.
* Chauffer la phase aqueuse B pour disperser le conservateur et le sel. Puis laisser refroidir à température ambiante.

### - Réalisation de l'émulsion :

* Peser les constituants de la phase A1 et les mélanger sous agitation Moritz à température ambiante puis ajouter A2, A3, A4 et A5 en prenant soin de bien homogénéiser entre chaque phase et d'augmenter la vitesse d'agitation si nécessaire.
* Mettre ensuite la totalité de la phase grasse A dans un bain d'eau froide et toujours sous agitation moritz, ajouter progressivement B en augmentant la vitesse d'agitation si nécessaire.
* Laisser sous agitation pendant 10mn

On a mesuré l'indice de transfert en présence de sébum les compositions des exemples 1 à 7.

### Résultats

| | Exemple 1 | Exemple 2 | Exemple 3 | Exemple 4 | Exemple 5 | Exemple 6 | Exemple 7 |
|---|---|---|---|---|---|---|---|
| % Régalite R 1100 | 100 | 80,00 | 76,19 | 66,67 | 61,90 | 51,43 | 50,00 |
| % Kraton G1657M | 0 | 20,00 | 23,81 | 33,33 | 38,10 | 48,57 | 50,00 |
| Push & Pull 4kg/Sec | 1,25 | 0 | 0 | 0 | 0 | 0 | 0 |
| Push & Pull 4kg/Eau | 1,25 | 0 | 0 | 0 | 0 | 0 | 0 |
| Push & Pull 4kg/Sueur | 1,5 | 0 | 0 | 0 | 0 | 0 | 0 |
| Push & Pull 4kg/Sébum | 2,5 | 3 | 0,5 | 1,5 | 1,25 | 1,5 | 1,5 |

L'exemple 1 montre que la présence de la résine copolymère styrène/méthyl styrène/indène hydrogéné Régalite R 1100 permet d'obtenir un dépôt ayant un indice de transfert en présence de sébum inférieur à 3.

Par ailleurs, les exemples 3 à 7 montrent que l'indice de transfert est inférieur à celui de l'exemple 1. L'association de copolymère styrène/méthyl styrène/indène hydrogéné (la résine) et de copolymère bloc styrène- éthylène/butylène- styrène (le copolymère bloc hydrocarboné) permet donc d'améliorer le non transfert, la tenue, de la composition par rapport à une composition contenant uniquement la résine Régalite R 1100.

Lorsque le rapport pondéral de copolymère styrène/méthyl styrène/indène hydrogéné / copolymère bloc styrène- éthylène/butylène- styrène (Régalite R 1100 / Kraton G1657M) est compris entre 1/1 et 3,5/1, l'indice de transfert en présence de sébum obtenu est inférieur à 2.

Le meilleur résultat de non transfert (indice de transfert en présence de sébum le plus faible) est observé pour un rapport pondéral de résine (Régalite R 1100) /(Kraton G1657M) proche de 3/1 (exemple 3).

Par ailleurs, les fonds de teint des exemples 1 à 7 appliqués sur la peau permettent d'obtenir un maquillage présentant une bonne tenue et un bon non transfert.

## Revendications

**1.** Composition de maquillage ou de soin de la peau comprenant une phase grasse liquide comprenant au moins une résine de poids moléculaire moyen en nombre inférieur ou égal à 10 000 g/mol, choisie parmi la colophane, les dérivés de colophane, les résines hydrocarbonées et leurs mélanges, la composition étant apte à former un dépôt ayant un indice de transfert en présence de sébum inférieur ou égal à 3.

**2.** Composition selon la revendication 1, **caractérisée par le fait que** le dépôt formé présente un indice de transfert en présence de sébum inférieur ou égal à 2, de préférence inférieur ou égal à 1,5.

**3.** Composition selon la revendication 1 ou 2, **caractérisée par le fait que** le dépôt formé présente un indice de transfert en présence de sébum inférieur ou égal à 1,5 , de préférence inférieur ou égal à 1.

**4.** Composition selon l'une quelconque des revendications précédentes, **caractérisée en ce que** la résine présente un poids moléculaire moyen en nombre allant de 250 à 10000 g/mol.

**5.** Composition selon la revendication précédente, **caractérisée en ce que** la résine présente un poids moléculaire moyen en nombre inférieur ou égal 5000 g/mol, notamment allant de 250 à 5000 g/mol, de préférence, inférieur ou égal à 2000 notamment allant de 250 à 2000 g/mol et préférentiellement inférieur ou égal à 1000 g/mol, notamment allant de 250 à 1000 g/mol.

**6.** Composition selon l'une quelconque des revendications précédentes, **caractérisée en ce que** les résines hydrocarbonées sont choisies parmi les résines hydrocarbonées indéniques, les résines aliphatiques de pentanediène, les résines mixtes de pentanediène et d'indène, les résines diènes des dimères de cyclopentanediène, les résines diènes des dimères de l'isoprène et leurs mélanges.

**7.** Composition selon l'une quelconque des revendications précédentes, **caractérisée en ce que** la résine est une résine hydrocarbonée indénique issue de la polymérisation de monomère indène et de monomère choisi parmi le styrène, le méthylindène, le méthylstyrène et leurs mélanges.

**8.** Composition selon la revendication précédente, **caractérisée en ce que** la résine hydrocarbonée indénique est hydrogénée.

**9.** Composition selon l'une quelconque des revendications 1 à 6, **caractérisée en ce que** la résine est une résine indénique choisie parmi les copolymères indène/méthylstyrène/styrène hydrogénés.

**10.** Composition selon l'une quelconque des revendications 1 à 6, **caractérisée en ce que** la résine est une résine aliphatique de pentanediène issue de la polymérisation du monomère 1,3-pentanediène (trans ou cis pipérylène) et de monomère choisi parmi l'isoprène, le butène le 2-méthyl-2-butène, le pentène, le 1, 4-pentanediène et leurs mélanges.

**11.** Composition selon l'une quelconque des revendications 1 à 6, **caractérisée en ce que** la résine est une résine mixte de pentanediène et d'indène.

**12.** Composition selon l'une quelconque des revendications 1 à 6, **caractérisée en ce que** la résine est une résine diène des dimères de cyclopentanediène issue de la polymérisation de premier monomère choisi parmi l'indène et le styrène, et de deuxième monomère choisi parmi les dimères du cyclopentanediène.

**13.** Composition selon l'une quelconque des revendications 1 à 6, **caractérisée en ce que** la résine est une résine diène des dimères de l'isoprène issue de la polymérisation d'au moins un monomère choisi parmi l'α-pinène, le β-pinène, le limonène, et leurs mélanges.

**14.** Composition selon l'une quelconque des revendications précédentes, **caractérisée en ce que** la résine est présente en une teneur allant de 0,1 à 30 % en poids, par rapport au poids total de la composition, de préférence allant de 0,3 à 15 % en poids, plus préférentiellement allant de 0,5 à 10 % en poids.

**15.** Composition selon l'une quelconque des revendications précédentes, **caractérisée en ce que** la phase grasse liquide comprend au moins une huile.

**16.** Composition selon la revendication précédente, **caractérisée en ce qu'**elle comprend au moins une huile volatile.

**17.** Composition selon la revendication précédente, **caractérisée en ce que** l'huile volatile est choisie parmi les huiles hydrocarbonées ayant de 8 à 16 atomes de carbone.

**18.** Composition selon les revendications 16 et 17, **caractérisée en ce que** l'huile volatile est présente dans la composition en une teneur allant de 2 à 80 % en poids, par rapport au poids total de la composition, de préférence allant de 5 à 70 % en poids.

**19.** Composition selon l'une quelconque des revendications précédentes, **caractérisée en ce que** la phase grasse liquide de la composition est présente dans la composition en une teneur totale allant de 2 % à 50 % en poids, par rapport au poids total de la composition, de préférence allant de 5 % à 40 % en poids.

**20.** Composition selon l'une quelconque des revendications précédentes, **caractérisée en ce que** la composition comprend au moins un copolymère bloc hydrocarboné.

**21.** Composition selon la revendication précédente, **caractérisée par le fait que** le copolymère bloc hydrocarboné est un copolymère amorphe formé par polymérisation de monomères de carbure éthylénique, ayant notamment une ou deux insaturations éthyléniques, ayant de 2 à 5 atomes de carbone.

**22.** Composition selon la revendication précédente, **caractérisée par le fait que** le copolymère bloc hydrocarboné est formé par polymérisation d'oléfine choisie parmi l'éthylène, le propylène, le butadiène, l'isoprène.

**23.** Composition selon l'une des revendications 20 à 22, **caractérisée par le fait que** le copolymère bloc hydrocarboné est un copolymère, éventuellement hydrogéné, à blocs styrène et à blocs éthylène/alkylène en C₃-C₄.

**24.** Composition selon l'une quelconque des revendications 20 à 23, **caractérisée par le fait que** le copolymère bloc hydrocarboné est choisi parmi les copolymères dibloc, éventuellement hydrogénés, de styrène-éthylène/propylène, de styrène-éthylène/butadiène, de styrène-étylène/butylène et les copolymères tribloc, éventuellement hydrogénés, de styrène-éthylène/butadiène-styrène, de styrène-butylène/éthylène-styrène de styrène-isoprène-styrène et de styrène-butadiène-styrène.

**25.** Composition selon l'une quelconque des revendications 20 à 24, **caractérisée par le fait que** le copolymère bloc hydrocarboné est un mélange de copolymère hydrogéné tribloc de styrène-butylène/éthylène-styrène et de copolymère dibloc de styrène-étylène/butylène.

**26.** Composition selon l'une quelconque des revendications 20 à 25, **caractérisée en ce que** le copolymère bloc hydrocarboné est présent dans la composition en une teneur allant de 0,1 % à 10 % en poids, par rapport au poids total de la composition, de préférence allant de 0,5 % à 5 % en poids, et plus préférentiellement allant de 1 % à 3 % en poids.

**27.** Composition selon l'une quelconque des revendications précédentes, **caractérisé en ce que** le fait que le rapport pondéral de la résine sur le copolymère bloc hydrocarboné va de 1/1 à 4/1

**28.** Composition selon la revendication précédente, **caractérisé en ce que** le rapport pondéral de la résine sur le copolymère bloc hydrocarboné va de 2,5/1 à 3,5/1.

**29.** Composition selon l'une quelconque des revendications précédentes, **caractérisée en ce qu'**elle comprend un agent épaississant polymérique des huiles choisi parmi :
- les polycondensats de type polyamide résultant de la condensation entre (α) au moins un acide choisi parmi les acides dicarboxyliques comprenant au moins 32 atomes de carbone et (β) un alkylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés ;
- les polymères siliconés du type :
1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications,
les groupes capables d'établir des interactions hydrogène pouvant être choisis parmi les groupes ester, amide, sulfonamide, carbamate, thiocarbamate, urée, uréthane, thiourée, oxamido, guanidino, biguanidino et leurs combinaisons,

**30.** Composition selon l'une quelconque des revendications précédentes, **caractérisé en ce que** la composition comprend au moins une phase pulvérulente choisie parmi les pigments, les nacres, et leurs mélanges.

**31.** Composition selon l'une quelconque des revendications précédentes, **caractérisé en ce que** la composition comprend une phase aqueuse en une teneur allant de 3% à 80% en poids, par rapport au poids total de la composition, de préférence allant de 5 à 60% en poids.

**32.** Composition selon l'une quelconque des revendications précédentes, **caractérisé en ce que** la composition comprend un ingrédient cosmétique choisi parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les sequestrants, les agents déodorants, les agents filmogènes, et leurs mélanges.

**33.** Composition de maquillage et/ou de soin de la peau comprenant une phase grasse liquide comprenant :
i) une résine de poids moléculaire moyen en nombre inférieur ou égal à 10000 g/mol, choisie parmi la colophane, les dérivés de colophane, les résines hydrocarbonées et leurs mélanges, et
ii) au moins un copolymère bloc hydrocarboné choisi parmi les copolymères dibloc de styrène-éthylène/propylène, de styrène-éthylène/butadiène, et les copolymères tribloc de styrène-éthylène/butadiène-styrène, de styrène-isoprène-styrène et de styrène-butadiène-styrène,
le rapport pondéral de la résine sur le copolymère bloc hydrocarboné allant de 1/1 à 4/1.

**34.** Composition de maquillage et/ou de soin selon la revendication précédente **caractérisée en ce que** le rapport pondéral de la résine sur le copolymère bloc hydrocarboné va de 2,5/1 à 3,5/1.

**36.** Composition de maquillage et/ou de soin selon la revendication 33 ou 34 **caractérisée en ce que** la résine est choisie parmi les copolymères indène/méthylstyrène/styrène hydrogéné.

**37.** Procédé non thérapeutique de maquillage ou de soin de la peau comprenant l'application sur la peau d'une composition selon l'une quelconque des revendications 1 à 36.

**38.** Procédé de maquillage et/ou de soin de la peau consistant à appliquer sur le peau une composition comprenant une phase grasse liquide comprenant une résine de poids moléculaire moyen en nombre inférieur ou égal à 10000 g/mol, choisie parmi la colophane, les dérivés de colophane, les résines hydrocarbonées et leurs mélanges, et au moins un copolymère bloc hydrocarboné choisi parmi les copolymères dibloc de styrène-éthylène/propylène, de styrène-éthylène/butadiène, et les copolymères tribloc de styrène-éthylène/butadiène-styrène, de styrène-isoprène-styrène et de styrène-butadiène-styrène, le rapport pondéral de la résine sur le copolymère bloc hydrocarboné allant de 1/1 à 4/1.

**39.** Procédé de maquillage et/ou de soin selon la revendication précédente **caractérisé en ce que** le rapport pondéral de la résine sur le copolymère bloc hydrocarboné va de 2,5/1 à 3,5/1.

**40.** Procédé de maquillage et/ou de soin selon la revendication 38 ou 39 **caractérisé en ce que** la résine est choisie parmi les copolymères indène/méthylstyrène/styrène hydrogéné.

**41.** Utilisation d'une composition selon l'une quelconque des revendications 1 à 36, pour obtenir un dépôt, notamment un maquillage sur la peau, ayant une bonne résistance au transfert, notamment en présence de sébum.
